# FASCICULE DE BREVET EUROPEEN

(11) **EP 1 466 591 B1**
(45) Date de publication et mention de la délivrance du brevet: **17.10.2007**
(21) Numéro de dépôt: 04290854.1
(22) Date de dépôt: 01.04.2004
(51) Int. Cl.: A61K 8/73, A61K 8/99, A61Q 19/00

(54) **Composition cosmétique lyophilisée comprenant de la gomme de Sclerotium et un actif d'origine marine**
Kosmetische Zusammensetzung erhalten durch Gefriertrocknung von Sclerotium-Gummi und Verbindungen maritimen Ursprungs
Lyophilised cosmetic composition comprising Sclerotium gum and a marine active compound

(30) Priorité: 02.04.2003 FR 0304102
(43) Date de publication de la demande: 13.10.2004
(73) Titulaire: Laboratoires BLC Thalgo Cosmetic, 83520 Roquebrune-sur-Argens (FR); LYOFAL, 13300 Salon de Province (FR)
(72) Inventeur: Pradines, Dominique, 83370 Saint-Aygulf (FR); Declomesnil-Vidal, Sophie, 13100 Aix-en-Provence (FR); Sirop, Jean-Claude, 83110 Sanary (FR)
(74) Mandataire: Zapalowicz, Francis

(56) Documents cités:
- WO-A-01/70271
- US-A- 5 425 939
- US-A- 6 071 541
- GRASSI M ET AL: "A study of the rheological behavior of scleroglucan weak gel systems" , CARBOHYDRATE POLYMERS, APPLIED SCIENCE PUBLISHERS, LTD. BARKING, GB, VOL. 29, NR. 2, PAGE(S) 169-181 XP004034389 ISSN: 0144-8617 * le document en entier *

## Description

La présente invention est relative à de nouvelles compositions cosmétiques ou dermatologiques obtenues par lyophilisation d'un mélange comprenant de la gomme de Sclerotium et au moins un composé actif d'origine marine.

Il est connu de lyophiliser des compositions cosmétiques afin d'améliorer la pénétration de composés actifs dans la peau du visage ou du corps.

On connaît notamment des compositions lyophilisées sous forme de feuilles de collagène bovin ou d'alginates alcalins.

Toutefois, ces compositions, après hydratation, soit doivent être retirées de la peau, soit sont relativement collantes et laissent des résidus à la surface de la peau.

La présente invention se propose de remédier à ces inconvénients.

En particulier, la Demanderesse vient de découvrir de façon surprenante que la lyophilisation d'un mélange comprenant de la gomme de Sclerotium et au moins un composé actif d'origine marine conduisait à des compositions qui étaient, après réhydratation, non collantes et filmogènes, et qui pénétraient rapidement dans les couches superficielles de l'épiderme après un massage très court, sans formation de résidus.

Elle a en outre constaté que ces compositions présentaient une action cosmétique rapide et efficace grâce à la bonne dispersion des composés actifs d'origine marine dans la gomme de Sclerotium servant de support.

Elle a également découvert que les compositions selon l'invention permettaient une bonne conservation des composés actifs, en particulier vis-à-vis de l'humidité.

Enfin, elle a constaté que ces compositions permettaient de délivrer facilement le composé actif d'origine marine.

D'autres objets de l'invention apparaîtront à la lecture de la description et des exemples qui suivent.

La présente invention a donc pour objet une composition cosmétique ou dermatologique, caractérisée en ce qu'elle est obtenue par lyophilisation d'un mélange comprenant de la gomme de Sclerotium et au moins un composé actif d'origine marine.

La gomme de Sclerotium est un homopolysaccharide constitué par une succession de monomères de glucose liés par des liaisons bêta 1-3, un résidu sur trois étant substitué par un monomère de glucose lié par une liaison bêta 1-6. Le poids moléculaire de la gomme de Sclerotium peut être compris entre 5 et 6 millions de daltons.

Grâce à la présence de la gomme de Sclerotium, la composition présente l'avantage de former une structure fine et expansée, associée à une excellente mémoire de forme, et de supporter la présence d'électrolytes susceptibles de se trouver dans le composé actif d'origine marine. En outre, la gomme de Sclerotium crée un réseau autour des particules de composé actif d'origine marine ce qui entraîne ainsi une très bonne dispersion du composé actif dans la composition. En outre, la gomme de Sclerotium est non ionique, ses interactions avec le composé actif d'origine marine sont donc très faibles ce qui facilite la libération du composé actif. Enfin, la gomme de Sclerotium forme, après réhydratation, un fluide pseudo plastique, ce qui augmente le pouvoir d'étalement de la composition par rapport à des gels de texturants naturels.

la gomme de Sclerotium peut être présente dans la composition lyophilisée selon l'invention à des teneurs comprises entre 50 et 80% en poids du poids total de la composition.

Pour optimiser l'obtention d'une structure expansée, poreuse, avec mémoire de forme et adhésion à la peau après réhydratation, la composition lyophilisée réhydratée présente de préférence une viscosité comprise entre 0,4 et 3,5 Pa.s. La réhydratation est menée avec de préférence de 0,1 à 0,3 g d'eau par cm² de la structure ci-dessus.

Le composé actif d'origine marine peut être choisi parmi les composés d'origine marine hydratants, restructurants, raffermissants, tensioactifs, antioxydants, adoucissants, anti-UV, dépigmentants, liporégulateurs, anti-acnéiques, anti-inflammatoires, et anti-vieillissement. Ils peuvent être de nature hydrophile ou lipophile. Le composé actif est protégé par le polysaccharide qui lui sert de support pour la lyophilisation.

Le composé actif d'origine marine peut être par exemple choisi parmi les extraits d'algues, les extraits de plantes, les extraits de collagène et les extraits d'élastine marine.

Le composé actif d'origine marine peut être présent dans la composition lyophilisée selon l'invention à des teneurs comprises entre 20 et 50% en poids du poids total de la composition.

La composition selon l'invention a un pH pouvant varier de 2 à 11.

Le mélange lyophilisé peut en outre comprendre des additifs cosmétiques usuels choisis notamment parmi les agents alcalinisants, les colorants, les pigments, les parfums, les conservateurs, les solubilisants, les tensioactifs et leurs mélanges.

Deux voies de préparation des compositions lyophilisées selon l'invention sont possibles.

Selon un premier procédé, le mélange comprenant la gomme de Sclerotium et le composé actif d'origine marine est déposé sur un support puis séché par lyophilisation. Le produit ainsi lyophilisé est ensuite découpé à la forme souhaitée puis conditionné à l'intérieur d'une pochette ou d'un blister étanche. On utilise de préférence un matériau de conditionnement étanche à l'oxygène et à la vapeur d'eau, tel qu'un matériau en matière plastique. Le conditionnement peut être réalisé sous vide ou sous atmosphère gazeuse neutre.

Selon un deuxième procédé, le mélange comprenant la gomme de Sclerotium et le composé actif d'origine marine est introduit dans une coque constituant un support et qui peut aussi servir de conditionnement du produit ultérieurement lyophilisé, et dont la forme géométrique peut être définie en fonction du corps et du visage auquel le produit est destiné. Dans ce cas, le mélange est coulé dans la coque et, au cours de la lyophilisation, le mélange prend la forme de la coque dans laquelle il a été lyophilisé, notamment par mémoire de forme.

Le produit ainsi lyophilisé selon ce deuxième procédé peut ensuite être démoulé puis conditionné à l'intérieur d'une pochette ou d'un blister. On utilise de préférence un matériau de conditionnement étanche à l'oxygène et à la vapeur d'eau, tel qu'un matériau en matière plastique. Le conditionnement peut être réalisé sous vide ou sous atmosphère gazeuse neutre.

Quel que soit le procédé utilisé, le produit lyophilisé peut prendre la forme d'un masque pour le visage, d'un contour de l'oeil, d'un contour des lèvres, de la zone entre le nez et la lèvre supérieure, du coin externe de l'oeil, des joues, du front ou de toute autre forme selon la zone du corps à traiter. La taille du produit lyophilisé selon l'invention est généralement comprise entre 0,25 et 650 cm², et de préférence entre 5 et 100 cm².

La lyophilisation est une technique bien connue de l'homme du métier. C'est une technique douce de séchage par le froid et le vide qui permet de conserver et de stabiliser la composition cosmétique ou dermatologique sans l'altérer.

La lyophilisation peut ainsi consister à refroidir le mélange jusqu'à une température inférieure à -10°C, de préférence comprise entre -20 et -80°C, pour congeler ou surgeler, de préférence pendant une durée d'au moins une heure, par exemple de 2 à 10 heures. On place ensuite le produit congelé sous un vide correspondant à une pression supérieure à 10 Pa, de préférence comprise entre 10 et 100 Pa, en maintenant le produit à une température inférieure à -5°C, de préférence comprise entre -15°C et -40°C, pour déshydrater le produit par sublimation, pendant une durée de préférence de plusieurs heures, notamment comprise entre 10 et 50 heures. On provoque ensuite une déshydratation secondaire (désorption) par chauffage progressif du produit jusqu'à une température finale comprise entre à 0 et 50°C, de préférence comprise entre 20 et 50°C, pendant une durée de préférence de plusieurs heures, de préférence de 5 à 30 heures.

Les exemples suivants sont destinés à illustrer l'invention.

### Exemples

On prépare les compositions lyophilisées suivantes (composés exprimés en pourcentages en poids du poids total de la composition)

### Exemple 1 : patch hydratant

| | |
|---|---|
| Gomme de Sclerotium | 60% |
| Extrait de Codium fragile | 30% |
| Parfum, solubilisant qsp | 100% |

### Exemple 2 : patch raffermissant, repulpant

| | |
|---|---|
| Gomme de Sclerotium | 60% |
| Extrait de Macrocystis pyrifera | 30% |
| Parfum, colorant, solubilisant qsp | 100% |

### Exemple 3 : patch rééquilibrant

| | |
|---|---|
| Gomme de Sclerotium | 60% |
| Extrait de Laminaria digitata | 30% |
| Parfum, solubilisant qsp | 100% |

### Exemple 4 : patch purifiant

| | |
|---|---|
| Gomme de Sclerotium | 60% |
| Extrait d'Asparagopsis armata | 30% |
| Solubilisant qsp | 100% |

Pour obtenir les compositions lyophilisées ci-dessus, on utilise des mélanges aqueux avec une proportion en eau supérieure afin d'obtenir, après lyophilisation, les teneurs en polysaccharides et en composés actif souhaitées. Chaque mélange est coulé à température ambiante dans une coque thermoformée en PVC sur une épaisseur moyenne de 3 mm. Après coulage, la coque est placée dans l'enceinte d'un lyophilisateur, puis le mélange est séché par lyophilisation. Les paramètres de l'étape de lyophilisation sont les suivants :
Congélation à -40°C pendant 5 heures
Sublimation à -20°C pendant 12 heures
Déshydratation secondaire à +30°C pendant 20 heures
Vide partiel sous pression de 40 Pa.

Après lyophilisation, la coque est fermée par un opercule en aluminium thermoscellé.

Après réhydratation, ces compositions sous forme de patchs forment une solution visqueuse ayant une viscosité de 0,5 Pa.s.

## Revendications

1. Composition cosmétique ou dermatologique lyophilisée, **caractérisée en ce qu**'elle comprend de la gomme de Sclerotium et au moins un composé actif d'origine marine.

2. Composition selon la revendication 1 **caractérisée en ce que** gomme de Sclerotium est présente dans la composition à des teneurs comprises entre 50 et 80% en poids du poids total de la composition.

3. Composition selon la revendication 1 ou 2, **caractérisée en ce que** le composé actif d'origine marine est choisi parmi les composés d'origine marine hydratants, restructurants, raffermissants, tensioactifs, antioxydants, adoucissants, anti-UV, dépigmentants, liporégulateurs, anti-acnéiques, anti-inflammatoires, et anti-vieillissement.

4. Composition selon la revendication 3, **caractérisée en ce que** le composé actif d'origine marine choisi parmi les extraits d'algues, les extraits de plantes, les extraits de collagène et les extraits d'élastine marine.

5. Composition selon l'une quelconque des revendications précédentes, **caractérisée en ce que** le composé actif d'origine marine est présent dans la composition à des teneurs comprises entre 20 et 50% en poids du poids total de la composition.

## Claims

1. Cosmetic or dermatological freeze-dried composition, **characterized in that** it comprises sclerotium gum and at least one active compound of marine origin.

2. Composition according to Claim 1, **characterized in that** the sclerotium gum is present in the composition in contents of between 50% and 80% by weight relative to the total weight of the composition.

3. Composition according to Claim 1 or 2, **characterized in that** the active compound of marine origin is chosen from moisturizing, restructuring, firming, surfactant, antioxidant, softening, anti-UV, depigmenting, liporegulating, antiacne, antiinflammatory and anti-ageing compounds of marine origin.

4. Composition according to Claim 3, **characterized in that** the active compound of marine origin is chosen from algal extracts, plant extracts, collagen extracts and marine elastin extracts.

5. Composition according to any one of the preceding claims, **characterized in that** the active compound of marine origin is present in the composition in contents of between 20% and 50% by weight relative to the total weight of the composition.

## Patentansprüche

1. Kosmetische oder dermatologische, gefriergetrocknete Zusammensetzung, **dadurch gekennzeichnet, dass** sie Sclerotiumgummi und mindestens einen Wirkstoff mariner Herkunft enthält.

2. Zusammensetzung nach Anspruch 1, **dadurch gekennzeichnet, dass** das Sclerotiumgummi in der Zusammensetzung in Mengenanteilen von 50 bis 80 Gew.-% des Gesamtgewichts der Zusammensetzung enthalten ist.

3. Zusammensetzung nach Anspruch 1 oder 2, **dadurch gekennzeichnet, dass** der Wirkstoff mariner Herkunft unter hydratisierenden Verbindungen, restrukturierenden Verbindungen, straffenden Stoffen, grenzflächenaktiven Stoffen, Antioxidantien, beruhigenden Stoffen, UV-Schutzmitteln, Depigmentierungsmitteln, Liporegulatoren, Wirkstoffen gegen Akne, entzündungshemmenden Wirkstoffen und Wirkstoffen gegen Alterung mariner Herkunft ausgewählt ist.

4. Zusammensetzung nach Anspruch 3, **dadurch gekennzeichnet, dass** der Wirkstoff mariner Herkunft unter den Algenextrakten, Pflanzenextrakten, Collagenextrakten und Elastinextrakten mariner Herkunft ausgewählt ist.

5. Zusammensetzung nach einem der vorhergehenden Ansprüche, **dadurch gekennzeichnet, dass** der Wirkstoff mariner Herkunft in der Zusammensetzung in Mengenanteilen von 20 bis 50 Gew.-% des Gesamtgewichts der Zusammensetzung enthalten ist.
